(19) Europäisches Patentamt / European Patent Office / Office européen des brevets

(11) **EP 4 368 573 A1**

(12) **EUROPEAN PATENT APPLICATION**
published in accordance with Art. 153(4) EPC

(43) Date of publication:
**15.05.2024 Bulletin 2024/20**

(21) Application number: **22837574.7**

(22) Date of filing: **29.06.2022**

(51) International Patent Classification (IPC):
*C01B 33/193* (2006.01)     *A61Q 17/04* (2006.01)
*A61K 8/25* (2006.01)

(52) Cooperative Patent Classification (CPC):
**A61K 8/25; A61Q 17/04; C01B 33/193**

(86) International application number:
**PCT/JP2022/026087**

(87) International publication number:
**WO 2023/282158 (12.01.2023 Gazette 2023/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.07.2021   JP 2021113071**

(71) Applicants:
- **AGC INC.**
  **Chiyoda-ku,**
  **Tokyo 1008405 (JP)**
- **AGC Si-Tech Co., Ltd.**
  **Fukuoka 808-0027 (JP)**

(72) Inventors:
- **KATAYAMA Hajime**
  **Tokyo 100-8405 (JP)**
- **KONDO Masashi**
  **Tokyo 100-8405 (JP)**
- **FUKUMOTO Kohta**
  **Tokyo 100-8405 (JP)**
- **TAKANO Yuriko**
  **Tokyo 100-8405 (JP)**
- **ARIMITSU Shinnosuke**
  **Kitakyushu-shi, Fukuoka 808-0027 (JP)**
- **MURAKAMI Takeshi**
  **Kitakyushu-shi, Fukuoka 808-0027 (JP)**
- **HIGASHI Kenji**
  **Kitakyushu-shi, Fukuoka 808-0027 (JP)**
- **YAMAZUMI Daisuke**
  **Kitakyushu-shi, Fukuoka 808-0027 (JP)**

(74) Representative: **Müller-Boré & Partner**
**Patentanwälte PartG mbB**
**Friedenheimer Brücke 21**
**80639 München (DE)**

(54) **LIGHT SCATTERING SILICA PARTICLES AND METHOD FOR PRODUCING LIGHT SCATTERING SILICA PARTICLES**

(57)     The present invention provides highly safe particles that have high scattering properties with respect to both visible light and ultraviolet light, while having a smooth texture. Light scattering silica particles according to the present invention have light scattering properties; and each of the particles internally has a plurality of hollow parts each having a closed pore structure. The light scattering silica particles have a 50% particle diameter of 1 $\mu$m to 500 $\mu$m and an average circularity of 0.8 or more. In cases where the light scattering silica particles are used, the reflectance A of a water cake having a silica amount per measurement cross-sectional area of 20 mg/cm2 is 30% or more at an ultraviolet wavelength of 310 nm.

*FIG. 1*

EP 4 368 573 A1

**Description**

TECHNICAL FIELD

The present invention relates to light-scattering silica particles and a method for producing light-scattering silica particles.

BACKGROUND ART

Light scattering occurs at an interface between two phases with different refractive indexes, and is a change in a traveling direction of light. Particles that cause such light scattering are used as fillers in various fields such as cosmetics, paints, and optical members.

A degree of light scattering varies depending on a wavelength of light, a size of particles, and a difference in refractive index between the particles and surrounding environment thereof.

In order to produce particles with a high light scattering property, for example, the size of the particles may be reduced to increase the number of interfaces, the particles may contain highly refractive materials such as titanium dioxide or zinc oxide, or hollow portions (void portions) may be formed inside the particles to increase the difference in refractive index with the surrounding environment.

On the other hand, when using particles as a filler, a feeling during use of the particles is also important, and especially in cosmetics, a good sense of touch such as slipperiness on skin is required.

A known method for imparting the light scattering property to particles and improving the sense of touch is to encapsulate a highly refractive material such as titanium dioxide or zinc oxide in spherical particles. For example, Patent Literature 1 proposes titanium dioxide-containing spherical silica having an average particle diameter of 1 μm to 10 μm, in which titanium dioxide having an average particle diameter of 0.01 μm to 0.5 μm is dispersed in a silica gel matrix at a content of 10 weight% to 60 weight% based on a total amount of $SiO_2$ and titanium dioxide.

CITATION LIST

PATENT LITERATURE

Patent Literature 1: JPH11-322324A

SUMMARY OF INVENTION

TECHNICAL PROBLEM

When the particles contain a highly refractive material such as titanium dioxide or zinc oxide, the scattering property for visible light can be increased, but since the highly refractive material absorbs ultraviolet rays, the scattering property for ultraviolet rays is low, and the light scattering property varies depending on the wavelength of the light. There are safety concerns about the particles containing metal elements such as titanium dioxide when the particles are used in applications such as cosmetics that may come into direct contact with skin or be taken into a body through eyes, mouth, or nose, and furthermore, since titanium dioxide and the like have a photocatalytic property, there is a risk that they may alter qualities of other raw materials.

Therefore, an object of the present invention is to provide particles that have both a light scattering property over a wide range of wavelengths and a good sense of touch, specifically, highly safe particles that have a high scattering property against both visible light and ultraviolet rays and have a smooth sense of touch.

SOLUTION TO PROBLEM

The present inventors conducted studies in view of the above-mentioned problem, and found that the above-mentioned problem could be solved by using spherical silica particles with a plurality of hollow portions each having a closed pore structure that are formed inside the particles, thereby completing the present invention.

The present invention relates to the following (1) to (16).

(1) Light-scattering silica particles having a light scattering property, the light-scattering silica particles including:

a plurality of hollow portions each having a closed pore structure inside the particle, in which
the light-scattering silica particles have a volume-based cumulative 50% particle diameter of 1 μm to 500 μm

and an average circularity of 0.80 or more, and
in use of the light-scattering silica particles, a reflectance A of a water cake containing 20 mg/cm$^2$ of silica per measurement cross-sectional area at an ultraviolet wavelength of 310 nm is 30% or more.

(2) The light-scattering silica particles according to (1), in which
the light-scattering silica particles have an oil absorption value of 100 cm$^3$/100 g or more.
(3) The light-scattering silica particles according to (1) or (2), in which
the hollow portions are defined by hollow particles.
(4) The light-scattering silica particles according to any one of (1) to (3), in which
each of the hollow portions have a size of 200 nm to 50 μm.
(5) The light-scattering silica particles according to any one of (1) to (4), in which
in use of the light-scattering silica particles, a reflectance B of a water cake containing 20 mg/cm$^2$ of silica per measurement cross-sectional area at a visible light wavelength of 600 nm is 30% or more.
(6) The light-scattering silica particles according to (5), in which
a difference (A - B) obtained by subtracting the reflectance B from the reflectance A is 3% or more.
(7) The light-scattering silica particles according to any one of (1) to (6), in which
one or two or more kinds selected from the group consisting of silicone, a silylating agent, a fatty acid having 12 or more carbon atoms and metal salts thereof, and a higher alcohol having 14 or more carbon atoms is supported on surfaces of the light-scattering silica particles.
(8) A method for producing light-scattering silica particles having a light scattering property, the method including:

dispersing hollow portion-forming particles in a silica precursor dispersion or a silica precursor solution;
performing an emulsification treatment to obtain droplets; and
solidifying the droplets.

(9) The method for producing light-scattering silica particles according to (8), in which
the hollow portion-forming particles are dispersed in the silica precursor dispersion or the silica precursor solution in a range of 0.1 mass% to 40 mass%.
(10) The method for producing light-scattering silica particles according to (8) or (9), in which
the hollow portion-forming particles are hollow particles.
(11) The method for producing light-scattering silica particles according to (10), in which the hollow particles are at least one of hollow silica particles and glass balloons.
(12) The method for producing light-scattering silica particles according to (10) or (11), in which a hollow portion inside each of the hollow particles has a size of 200 nm to 50 μm.
(13) The method for producing light-scattering silica particles according to (8) or (9), in which
the hollow portion-forming particles are template particles, and after the droplets are solidified, the template particles are removed, followed by firing at 800°C to 1300°C.
(14) The method for producing light-scattering silica particles according to (13), in which
the template particles have a size of 200 nm to 50 μm.
(15) The method for producing light-scattering silica particles according to any one of (8) to (14), in which
one or two or more kinds selected from the group consisting of silicone, a silylating agent, a fatty acid having 12 or more carbon atoms and metal salts thereof, and a higher alcohol having 14 or more carbon atoms is attached to surfaces of particles obtained by solidifying the droplets.
(16) A cosmetic including the light-scattering silica particles according to any one of (1) to (7).

ADVANTAGEOUS EFFECTS OF INVENTION

The light-scattering silica particles according to the present invention have a light scattering property for both visible light and ultraviolet rays, and also have a smooth sense of touch when touched. Since the hollow portions inside the particle each have a closed pore structure, liquids such as water and oil do not infiltrate into the hollow portions, and the light-scattering silica particles can exhibit the light scattering property regardless of whether being used in a dry state or a wet state. Furthermore, since the light-scattering silica particles are made from silica, safety thereof is high. Therefore, the light-scattering silica particles can be suitably used in cosmetics.

BRIEF DESCRIPTION OF DRAWINGS

[FIG. 1] FIG. 1 is a photographic diagram showing a scanning electron microscope image (SEM image) of light-scattering silica particles obtained in Example 2.

[FIG. 2] FIG. 2 is a photographic diagram showing a scanning electron microscope image (SEM image) of silica particles obtained in Example 6.

[FIG. 3] FIG. 3 is a photographic diagram showing optical microscope images of light-scattering silica particles obtained in Example 3, and silica particles of Examples 4 and 6.

[FIG. 4] FIG. 4 is a graph showing shielding properties of the light-scattering silica particles obtained in Example 3 and the porous spherical silica particles of Example 4.

[FIG. 5] FIG. 5 is a graph showing a reflectance spectrum of the light-scattering silica particles obtained in Example 3 immediately after preparation of a 1% water slurry.

DESCRIPTION OF EMBODIMENTS

The present invention will be described below, and the present invention is not limited by exemplifications in the following description.

Note that in the present description, "mass" is synonymous with "weight".

<Light-scattering Silica Particles>

The light-scattering silica particles according to the present invention include a plurality of hollow portions each having a closed pore structure inside the particle, have a volume-based cumulative 50% particle diameter of 1 $\mu$m to 500 $\mu$m, and have an average circularity of 0.80 or more. In use of the light-scattering silica particles, a reflectance A of a water cake containing 20 mg/cm$^2$ of silica per measurement cross-sectional area at an ultraviolet wavelength of 310 nm is 30% or more.

The "hollow portions each having a closed pore structure" existing inside the particles refer to voids that exist independently inside the particles and do not communicate with outside.

By observing particle powder immersed in boiled linseed oil using an optical microscope (for example, Morphologi 4, manufactured by Malvern Panalytical Ltd), it can be confirmed whether the particles include hollow portions inside. In the case where there are hollow portions inside the particles, scatterers with a contrast different from surrounding is observed.

Whether the hollow portions inside the particles each have a closed pore structure or not (whether an open pore structure or not) can be confirmed by immersing the particles in a liquid such as water, and in the case where no liquid infiltrates the hollow portions, it can be said that the hollow portions each have a closed pore structure. In the case where the liquid infiltrates the hollow portions, a refractive index of the hollow portions approaches that of the surrounding environment (silica constituting the particles and a dispersion medium such as water or oil), and the particles become translucent. In the case where an air phase in the hollow portions is maintained, the hollow portions reflect light and appear white, in the same state as presence of air bubbles in a liquid.

Since the hollow portions encapsulated in the light-scattering silica particles according to the present invention each have a closed pore structure, liquids do not infiltrate into the hollow portions, and therefore, the light-scattering silica particles can exhibit the light scattering property regardless of whether being used in a dry state or a wet state.

The light-scattering silica particles according to the present invention are particles including silica ($SiO_2$) as a main component. In the present description, "including silica as a main component" specifically means that a concentration of silica is 80 mass% or more, preferably 90 mass% or more, and more preferably 95 mass% or more. An upper limit is theoretically 100 mass%. A content of silica constituting the light-scattering silica particles is preferably less than 100 mass%, and more preferably 99 mass% or less. Examples of residual components include an alkali metal oxide, and carbon.

In porous silica, in the case where a pore diameter is sufficiently smaller than a wavelength of light, light is scattered at an interfaces between an outer surfaces of the silica particles and air, so that the more interfaces between the outer surfaces and air that is not inside the pore, the more light will be scattered. Therefore, since there is a plurality of hollow portions (voids) having a size equal to or larger than a wavelength of ultraviolet rays inside the particle, the hollow portions and silica layers around the hollow portions act as the interface that scatters light, and in this way, the light scattering property is improved, and as a result, a concealing property is improved.

A size of the hollow portion encapsulated in the light-scattering silica particle is preferably 200 nm to 50 $\mu$m. Note that the size of the hollow portion refers to a length of a long axis of the hollow portion, and a diameter of the smallest circumscribed circle circumscribing the hollow portion observed by SEM or TEM is defined as the size of the hollow portion. By encapsulating hollow portions each having a size of 200 nm or more in the particles, light scattering can be caused, and considering the size (particle diameter) of the light-scattering silica particles that can be produced, the size of the hollow portion is preferably 50 $\mu$m or less.

The size of the hollow portion inside the particle is more preferably 230 nm or more, and particularly preferably 250 nm or more. The smaller the size of the hollow portion, the more hollow portions can be encapsulated in the particles, and therefore, the number of scattering interfaces increases. Accordingly, the size of the hollow portion is more preferably 10 $\mu$m or less, and still more preferably 1 $\mu$m or less, and particularly preferably 0.5 $\mu$m or less. Note that in the case where the size of the hollow portion is 200 nm to 400 nm, ultraviolet rays are likely to be scattered more preferentially than visible light.

The number of hollow portions may be two or more in one particle, and can be adjusted as appropriate depending on the size of the light-scattering silica particles and the size of the hollow portion.

A volume ratio of the plurality of hollow portions in the light-scattering silica particles is preferably 40% or less of the volume of the light-scattering silica particles. The number of interfaces can be increased by increasing the number of hollow portions inside the particles, but even if there are too many hollow portions, the scattering of light will reach equilibrium and the scattering effect will not change. The volume ratio of the plurality of hollow portions is more preferably 30% or less, still more preferably 20% or less, and particularly preferably 10% or less. A lower limit thereof is not particularly limited as long as the effects of the present invention can be obtained, and the lower limit thereof is preferably 0.1% or more, more preferably 0.5% or more, and still more preferably 1% or more. A specific gravity of the particles can also be reduced by increasing the volume ratio of the hollow portions inside the particles.

The volume ratio of the hollow portions in the light-scattering silica particles is expressed as a percentage obtained by dividing total volume occupied by the hollow portions by a sum of a volume occupied by silica and a total volume occupied by the hollow portions, and can be obtained as follows.

The volume ratio of the hollow portions is determined by calculation using a density (g/cm$^3$) of the light-scattering silica particles measured using argon (Ar) gas and a true density (2.2 g/cm$^3$) of silica. Assuming that the measured density is $\rho$ (g/cm$^3$), the volume ratio of the hollow portion is 100 - 100 $\times$ $\rho$/2.2 (%).

Note that a silica layer that does not transmit Ar gas can be said to be a silica layer that does not substantially transmit water. In the case where a proportion of the hollow portions is large, it is also possible to estimate the volume ratio of the hollow portions based on the above density difference.

In the present invention, the silica surrounding the hollow portion may be porous silica or non-porous silica. When used as a cosmetic, porous silica absorbs sebum and sweat, thereby improving the feeling during use. Since the hollow portions in the light-scattering silica particles according to the present invention each have a closed pore structure, the light scattering property does not change even after the particles absorb sebum or sweat. In the case where slipperiness is important, non-porous silica may be preferable even for cosmetic applications. In the case where the light-scattering silica particles are used as a filler for resin, if the silica is porous, it may cause bubbles or increase in viscosity, and therefore, it is preferable to use non-porous silica.

In the present invention, a pore volume of the light-scattering silica particles is preferably 4 cm$^3$/g or less. Here, the pore volume refers to a pore volume derived from pore with a pore diameter of 1 nm to 100 nm. The pores (voids) around the hollow portion reduce an apparent density of the particles. That is, in the case where the silica is porous silica, the light-scattering silica particles become light particles and have improved sense of touch. In the case where the pore volume is too large, particle strength may decrease and applications thereof will be limited, and the pore volume is preferably 4 cm$^3$/g or less.

The pore volume is more preferably 3 cm$^3$/g or less, and still more preferably 2.5 cm$^3$/g or less. A lower limit of the pore volume in the light-scattering silica particles is not particularly limited, and in the case of porous silica, the pore volume is preferably 0.1 cm$^3$/g or more, more preferably 0.8 cm$^3$/g or more, and still more preferably 1.0 cm$^3$/g or more.

Note that the pore volume can be calculated using the BJH method by a nitrogen adsorption method.

The light-scattering silica particles according to the present invention preferably have an oil absorption value of 100 cm$^3$/100 g or more. In the case where the oil absorption value is 100 cm$^3$/100 g or more, the apparent density can be reduced and the particles become light particles, and a high concealing property can be maintained even in the case where some sebum is absorbed, so that the particles can be suitably used for cosmetic applications.

The oil absorption value is more preferably 150 cm$^3$/100 g or more, still more preferably 200 cm$^3$/100 g or more, and particularly preferably 250 cm$^3$/100 g or more. Although an upper limit of the oil absorption value is not particularly limited, from the viewpoint of particle strength, the oil absorption value is preferably 1000 cm$^3$/100 g or less, more preferably 800 cm$^3$/100 g or less, and still more preferably 600 cm$^3$/100 g or less.

The oil absorption value can be measured in accordance with JIS K 5101 (2004). Specifically, boiled linseed oil is added to a sample while the entire sample is kneaded until the sample becomes a lump. The oil absorption value is represented by a volume of the boiled linseed oil per 100 g of the sample when the entire sample becomes a lump. Hereinafter, the oil absorption value obtained by this measurement method will simply be referred to as the oil absorption value.

The light-scattering silica particles according to the present invention preferably have a specific surface area obtained based on the nitrogen adsorption method of 100 m$^2$/g or more. In the case where the specific surface area is

100 m$^2$/g or more, active sites can be increased when the particles are used as catalyst supports.

The specific surface area is more preferably 200 m$^2$/g or more, still more preferably 250 m$^2$/g or more, particularly preferably 300 m$^2$/g or more, and an upper limit thereof is not particularly limited.

Note that the specific surface area can be calculated using the BET theory after obtaining an adsorption isotherm by the nitrogen adsorption method.

The size of the light-scattering silica particles is calculated as a 50% volume equivalent particle diameter using a laser diffraction particle size distribution analyzer (for example, MT3300EXII manufactured by MicrotracBEL Corp.). Hereinafter, the volume-based cumulative 50% particle diameter of the particles will also be simply referred to as "50% particle diameter".

The 50% particle diameter ($D_{50}$) of the light-scattering silica particles is in a range of 1 μm to 500 μm. In the case where the 50% particle diameter ($D_{50}$) of the light-scattering silica particles is within the above range, dispersibility becomes better and the sense of touch becomes smoother, resulting in an improved feeling during use, when the particles are used as fillers. From the viewpoint of the feeling during use of cosmetics when the light-scattering silica particles are used as cosmetic materials, the 50% particle diameter ($D_{50}$) is preferably 2 μm or more, more preferably 3 μm or more, and still more preferably 4 μm or more. Moreover, from the viewpoint of handleability as a filler, an upper limit of the 50% particle diameter is preferably 100 μm or less, more preferably 50 μm or less, and still more preferably 20 μm or less.

An average circularity of the light-scattering silica particles is 0.80 or more. In the case where the average circularity is 0.80 or more, a shape of the particle becomes close to a spherical shape, so that the sense of touch on the skin becomes better and the particles can be suitably used for various purposes.

The average circularity of the silica particles is more preferably 0.90 or more, and still more preferably 0.95 or more. An upper limit of the circularity is not particularly limited, and the circularity is most preferably 1.

Note that the circularity can be calculated by obtaining an area and a perimeter of a particle with an image obtained by a particle image analyzer (for example, Morphologi 4 manufactured by Malvern Panalytical Ltd) by using image analysis software attached to the above analyzer and applying the results to the following equation. The average circularity is an average value of 30,000 particles.

$$Circularity = perimeter\ of\ circle\ with\ equal\ projected\ area/perimeter\ of\ particle$$

Perimeter of circle with equal projected area: observing a particle from directly above, obtaining an area of a shadow of the particle reflected on a plane below, and calculating a circle having an area equal to this area, so as to obtain a length of an outline of the circle

Perimeter of particle: observing the particle from directly above to obtain a length of an outline of the shadow of the particle reflected on the plane below

In the present invention, it is preferable that the hollow portions in the light-scattering silica particles are defined by hollow portion-forming particles. In the present description, the term "hollow portion-forming particles" refers to particles that are added to raw materials in order to form the hollow portions during production of the light-scattering silica particles. By defining the hollow portions with the hollow portion-forming particles, it is easy to make the hollow portion into a closed pore structure, and when the light-scattering silica particles come into contact with a liquid such as water or oil, the liquid can be prevented from infiltrating the hollow portions, and a shielding property caused by light scattering can be maintained. Note that in the case where hollow portion-forming particles made of organic matter are used, after forming the particles encapsulating the hollow portion-forming particles and then removing the hollow portion-forming particles by firing or dissolving treatment, the closed pore structure can be defined by performing heat treatment at a high temperature to cover the hollow portions with a dense phase.

Examples of the hollow portion-forming particles include hollow particles and template particles.

Examples of the hollow particles include hollow silica particles, glass balloons, and resin hollow particles. Moreover, a size of a hollow portion of the hollow particle roughly matches the size of the hollow portion of the light-scattering silica particle. By using the hollow particles as the hollow portion-forming particles, it is possible to form a closed pore hollow portion without a firing treatment, and since porous portions are not lost during firing, it is possible to provide light-scattering particles with a high oil absorption value.

Examples of the template particles include resin particles and acid-soluble inorganic particles, and examples of the acid-soluble inorganic particles include magnesium carbonate, magnesium hydroxide, calcium carbonate, and calcium hydroxide. A size of the template particle roughly matches the size of the hollow portion of the light-scattering silica particle.

Among these, it is particularly preferable that the hollow portions are defined by hollow particles from the viewpoint of easily defining the hollow portions into a dense closed pore structure.

The light-scattering silica particles according to the present invention may contain a surface treatment agent supported on surfaces thereof. By supporting the surface treatment agent, the particles are imparted with hydrophobicity and can be uniformly dispersed in resin or a solvent.

Examples of the surface treatment agent include silicone, silylating agents, fatty acids having 12 or more carbon atoms and metal salts thereof, and higher alcohols having 14 or more carbon atoms, and the surface treatment agent is selected from one type or a combination of two or more types of these.

Examples of silicone include dimethyl silicone, methyl hydrogen silicone, methylphenyl silicone, alkyl-modified silicone, and silicone oils such as fatty acid-modified silicone oil, polyether-modified silicone oil, alkoxy-modified silicone oil, carbinol-modified silicone oil, amino-modified silicone oil, fluorine-modified silicone oil, and terminal-reactive silicone oil.

Examples of the silylating agent include chlorosilanes such as dimethyldichlorosilane, trimethylchlorosilane, phenyldimethylchlorosilane, t-butyldimethylchlorosilane, and vinyldimethylchlorosilane; alkoxysilanes such as methyltrimethoxysilane, dimethyldimethoxysilane, phenyltrimethoxysilane, n-butyltrimethoxysilane, hexyltrimethoxysilane, octyltrimethoxysilane, decyltrimethoxysilane, dodecyltrimethoxysilane, vinyltrimethoxysilane, methyltriethoxysilane, dimethyldiethoxysilane, phenyltriethoxysilane, i-butyltriethoxysilane, decyltriethoxysilane, vinyltriethoxysilane, $\gamma$-meth-acryloxypropyltrimethoxysilane, $\gamma$-glycidoxypropyltnmethoxysilane, $\gamma$-mercaptopropyltnmethoxysilane, and N-phenyl-$\gamma$-aminopropyltrimethoxysilane; and silazane such as hexamethyldisilazane, hexaethyldisilazane, hexacyclohexyldisilazane, hexaphenyldisilazane, divinyltetramethyldisilazane, and dimethyltetravinyldisilazane.

Examples of the fatty acids having 12 or more carbon atoms and metal salts thereof include long-chain fatty acids such as lauric acid, tridecylic acid, dodecylic acid, myristic acid, palmitic acid, stearic acid, oleic acid, linoleic acid, arachidonic acid, and behenic acid, and salts of the above fatty acids and metals such as zinc, iron, magnesium, aluminum, calcium, sodium, and lithium.

Examples of the higher alcohol having 14 or more carbon atoms include myristyl alcohol, cetyl alcohol (cetanol), 2-hexyldecanol, stearyl alcohol, cetostearyl alcohol, oleyl alcohol, linoleyl alcohol, linolenyl alcohol, isostearyl alcohol, chimyl alcohol, arachidyl alcohol, octyldodecanol, ethylene glycol monostearate, stearic acid monoethanolamide, glycerol monostearate, selachyl alcohol, batyl alcohol, behenyl alcohol, decyltetradecanol, and camerbil alcohol.

Among the above surface treatment agents, in the light-scattering silica particles used in the cosmetics field, from the viewpoint of being a naturally derived from raw material and being kind to environment and human bodies, it is preferable to support a fatty acid having 12 or more carbon atoms and a metal salt thereof, or a higher alcohol having 14 or more carbon atoms, and more preferable to support a higher alcohol having 14 or more carbon atoms.

The higher alcohol having 14 or more carbon atoms is preferably a higher alcohol having 14 to 36 carbon atoms, from the viewpoint of being easily available industrially and imparting water repellency to the surfaces of the particles. The number of carbon atoms of the higher alcohol is more preferably 16 or more, still more preferably 18 or more, particularly preferably 20 or more, and is more preferably 30 or less, still more preferably 28 or less, and particularly preferably 24 or less.

The higher alcohol may be linear or branched, and may be saturated or unsaturated.

The number of hydroxyl groups in the higher alcohol having 14 or more carbon atoms may be one or more. Practically, the number of hydroxyl groups is preferably five or less, and more preferably three or less.

The higher alcohol having 14 or more carbon atoms may include a long-chain alkyl group, which may be unsaturated, and a hydroxyl group. However, a linking group may be present between the long-chain alkyl group, which may be unsaturated, and the hydroxyl group. Examples of the linking group include an ester bond, an ethereal oxygen atom, and an amide bond.

In the case where the light-scattering silica particles contain a surface treatment agent, a supported amount of the surface treatment agent is preferably 1 $\mu$mol/m$^2$ to 6 $\mu$mol/m$^2$ per unit specific area of the silica particles. In the case where the supported amount of the surface treatment agent is within the above range, sufficient hydrophobicity can be imparted.

The supported amount of the surface treatment agent is more preferably 1.2 mol/m$^2$ or more, and still more preferably 1.5 $\mu$mol/m$^2$ or more, and more preferably 5 $\mu$mol/m$^2$ or less, and still more preferably 4 $\mu$mol/m$^2$ or less.

The light-scattering silica particles according to the present invention is produced so that in use of the light-scattering silica particles, a reflectance A of a water cake containing 20 mg/cm$^2$ of silica per measurement cross-sectional area at an ultraviolet wavelength of 310 nm is 30% or more. In the case where the reflectance A measured as above is 30% or more, the particles have a light scattering property against ultraviolet rays, and it can be seen that since water does not infiltrate the hollow portions of the light-scattering silica particles, the light scattering property can be exhibited in both dry and wet states.

The reflectance A is preferably 35% or more, more preferably 40% or more, and still more preferably 45% or more. In order to exhibit the expected effects with a small amount, the reflectance A is preferably as high as possible, and an upper limit thereof is not particularly limited.

The light-scattering silica particles according to the present invention is preferably produced so that in use of the light-scattering silica particles, a reflectance B of a water cake containing 20 mg/cm$^2$ of silica per measurement cross-sectional area at a visible light wavelength of 600 nm is 30% or more. In the case where the reflectance B measured as above is 30% or more, the particles have a light scattering property against visible light, and it can be seen that since water does not infiltrate the hollow portions of the light-scattering silica particles, the light scattering property can be exhibited in both dry and wet states.

The reflectance B is preferably 35% or more, and more preferably 40% or more. Since the reflectance B is an index indicating whiteness and the concealing property of the particles, the reflectance B is preferably as high as possible, and an upper limit thereof is not particularly limited.

Note that in the present description, the reflectance is a relative reflectance when a reflectance of a standard white plate (barium sulfate) is assumed as 100%, and refers to a reflectance when total reflected light (a sum of regularly reflected light and diffusely reflected light) is measured using an integrating sphere. The reflectance can be obtained by measuring the silica water cake using an ultraviolet-visible light-near infrared spectrophotometer (for example, UV-3100PC, manufactured by SHIMADZU CORPORATION) as a measuring device.

Specifically, water is added to a powder of the light-scattering silica particles, followed by mixing, to produce a silica water cake, and the water cake is packed into a powder sample cell so that an amount of silica per measurement cross-sectional area is 20 mg/cm$^2$ to 30 mg/cm$^2$, and a reflectance (reflectance a) of the water cake at wavelengths of 310 nm and 600 nm is measured using the ultraviolet-visible light-near infrared spectrophotometer. Then, a reflectance (reflectance b) at wavelengths of 310 nm and 600 nm is measured without packing the cell with the sample, and an interpolated value of the reflectance a and the reflectance b is calculated as the reflectance when the amount of silica per measurement cross-sectional area is 20 mg/cm$^2$.

A difference (A - B) obtained by subtracting the reflectance B at a visible light wavelength of 600 nm from the reflectance A at an ultraviolet wavelength of 310 nm is preferably 3% or more. In the case where the difference A - B is 3% or more, an amount of reflected light with short wavelengths becomes large, thereby giving the reflected light a slightly bluish tinge, and a sense of transparency.

The difference A - B is more preferably 5% or more, and still more preferably 7% or more. In the case where blueness is desired to be emphasized, the higher the difference A - B is, the better, and an upper limit thereof is not particularly limited.

It is preferable that the light-scattering silica particles according to the present invention maintain the same reflectance A and reflectance B as measured above even after being kept in the water cake for one week.

In the light-scattering silica particles according to the present invention, it is preferable that when a mixed powder is obtained by mixing reddish brown iron (III) oxide powder (for example, Guaranteed reagent produced by KANTO CHEMICAL CO., INC.) with an a' value of 14 or more and 15 or less in the L'a'b' color system at a ratio of 40 mass% to the light-scattering silica particles, an a' value of the mixed powder is 6.0 or less. Note that the L'a'b' color system was standardized by the Commission Internationale de l'Eclairage (CIE) in 1976.

With the above measurement method, the light scattering property of the light-scattering silica particles in a dry state can be evaluated. In the case where the a' value is 6.0 or less, it can be evaluated that the scattering property against visible light in a dry powder is high, and the shielding property is excellent.

The a' value is more preferably 5.0 or less, still more preferably 4.5 or less, and particularly preferably 4.0 or less, and since the smaller the a' value, the better, a lower limit thereof is preferably 0.

Note that the a' value of the powder can be measured, for example, using a spectrocolorimeter (for example, SE7700, manufactured by NIPPON DENSHOKU INDUSTRIES CO., LTD.). In the measurement using a spectrocolorimeter, the mixed powder is added to a thickness of 7.5 mm or more and measured.

It is preferable that the light-scattering silica particles according to the present invention have both a dynamic friction coefficient and a static friction coefficient of 0.60 or less. In the case where the dynamic friction coefficient is 0.60 or less, the particles slide with low resistance, and therefore, when used in cosmetics, the sense of touch on the skin is improved, and thus a smooth sliding sensation can be exhibited. The dynamic friction coefficient is more preferably 0.55 or less, still more preferably 0.50 or less, and particularly preferably 0.40 or less. Although a lower limit of the dynamic friction coefficient is not particularly limited, the dynamic friction coefficient is preferably 0.05 or more, more preferably 0.10 or more, and still more preferably 0.15 or more.

In the case where the static friction coefficient is 0.60 or less, the particles begin to slide with low resistance, and therefore, when used in cosmetics, the sense of touch on the skin is improved. The static friction coefficient is more preferably 0.50 or less, and still more preferably 0.40 or less. Although a lower limit of the static friction coefficient is not particularly limited, the static friction coefficient is more preferably 0.01 or more, still more preferably 0.05 or more, and particularly preferably 0.10 or more.

Note that the dynamic friction coefficient can be measured using a static and dynamic friction measuring device (for example, "TL201Tt" manufactured by Trinity-Lab.Inc.). Specifically, a urethane artificial finger is used as a contactor and artificial leather is used as a coating substrate, and hydrophobic silica particles are applied to the artificial leather so that an adhesion amount in terms of a bulk volume per unit area is 0.8 μL/cm$^2$, a friction coefficient is measured by causing the measuring device to operate with a load of 30 gf and a scanning distance of 40 mm, and an average value in a range of 1,000 msec to 4,000 msec is employed as a dynamic friction coefficient.

Regarding the static friction coefficient, a friction coefficient is measured by the same method as above, and a maximum value within a range of 0 msec to 1,000 msec is employed as the static friction coefficient.

<Method for Producing Light-scattering Silica Particles>

A method for producing the light-scattering silica particles according to the present invention includes dispersing the hollow portion-forming particles in a silica precursor dispersion or a silica precursor solution, performing an emulsification treatment to obtain droplets, and solidifying the droplets.

First, a dispersion liquid in which the hollow portion-forming particles are dispersed in a silica precursor dispersion or a silica precursor solution is used as a dispersed phase, and an organic phase containing a surfactant is used as a continuous phase, and the dispersed phase and the continuous phase are mixed and stirred to obtain an emulsion.

By stirring and emulsifying the dispersed phase and the continuous phase, a water-in-oil (W/O) emulsion is obtained. The dispersed phase becomes droplets in the continuous phase, and the hollow portion-forming particles are present in the droplets of the silica precursor dispersion or the silica precursor solution.

The silica precursor dispersion or the silica precursor solution is a solution in which a silica precursor is dispersed or dissolved in a solvent. Examples of the silica precursor include sodium silicate, potassium silicate, lithium silicate, tetramethoxysilane, tetraethoxysilane, and tetrapropoxysilane. Examples of the solvent for dispersing or dissolving the silica precursor include water and lower alcohols such as methanol, ethanol, propanol, and butanol.

A concentration of the silica precursor is preferably in a range of 3 mass% to 35 mass% in terms of $SiO_2$ concentration converted to $SiO_2$. In the case where the $SiO_2$ conversion concentration of the silica precursor dispersion or the silica precursor solution is within the above range, the light-scattering silica particles can be produced with high production efficiency.

The $SiO_2$ conversion concentration of the silica precursor dispersion or the silica precursor solution is more preferably 5 mass% or more, still more preferably 7 mass% or more, and particularly preferably 10 mass% or more, from the viewpoint of high productivity and obtaining particles with high sphericity. From the viewpoint of easily obtaining particles with a small particle diameter, the $SiO_2$ conversion concentration is more preferably 30 mass% or less, still more preferably 25 mass% or less, and particularly preferably 20 mass% or less.

In the case where a sodium silicate aqueous solution is used as the silica precursor dispersion or the silica precursor solution, a ratio of a silica content to sodium in the sodium silicate aqueous solution, which is $SiO_2/Na_2O$ (molar ratio), is preferably 2 to 4. The sodium silicate aqueous solution having the $SiO_2/Na_2O$ (molar ratio) in the above range is easily available, and examples thereof include No. 3 sodium silicate (manufactured by AGC Si-Tech Co., Ltd., $SiO_2/Na_2O = 3$), No. 2 sodium silicate (manufactured by AGC Si-Tech Co., Ltd., $SiO_2/Na_2O = 2.5$), and No. 1 sodium silicate (manufactured by AGC Si-Tech Co., Ltd., $SiO_2/Na_2O=2$).

The hollow portion-forming particles are not particularly limited as long as they can define the hollow portions inside the light-scattering silica particles, and examples thereof include hollow particles with hollow portions inside or template particles without hollow portions and that can be removed later by firing or dissolving.

For the hollow particles, a substance that does not dissolve in the silica precursor dispersion or the silica precursor solution and does not absorb ultraviolet light is used. The hollow particles are encapsulated in the light-scattering silica particles, and the hollow particles define the hollow portions of the light-scattering silica particles.

Examples of the hollow particles include hollow silica particles, glass balloons, and resin hollow particles. Examples of the resin hollow particles include hollow particles formed from silicone resin or acrylic-styrene resin. These substances may be used alone or in combination of two or more kinds thereof.

For the template particles, a substance that does not dissolve in the sodium silicate aqueous solution but can be decomposed by heat or dissolved by acid is used. The template particles are removed in a producing step of the light-scattering silica particles, and the voids remaining after the removal define the hollow portions of the light-scattering silica particles.

Examples of thermally decomposable template particles that can be decomposed by heat include resin particles such as polypropylene, acrylic, styrene, and silicone. Examples of acid-soluble template particles that can dissolve in acid include magnesium hydroxide, magnesium carbonate, calcium hydroxide, and calcium carbonate. These substances may be used alone or in combination of two or more kinds thereof. Among these, at least one kind selected from the group consisting of magnesium hydroxide and magnesium carbonate is preferred from the viewpoint that template particles can be dissolved and removed using sulfuric acid. Sulfuric acid is a non-volatile acid and is more preferable than volatile acids such as hydrochloric acid, nitric acid, and hydrofluoric acid from the viewpoint of safety and equipment protection.

Among these, from the viewpoint of simplicity of producing steps and ease of making closed pores, it is preferable to use hollow particles covered with a dense shell as the hollow portion-forming particles, and at least one of hollow silica particles and glass balloons is more preferred.

The size of the hollow portion-forming particle may be appropriately selected in consideration of the size (particle diameter) of the light-scattering silica particle, a desired light scattering property, and the like.

Specifically, in the case where the hollow portion-forming particles are hollow particles, it is preferable to use hollow particle in which the size of the hollow portion inside the hollow particle is in a range of 200 nm to 50 $\mu$m. The size of the hollow portion is more preferably 230 nm or more, still more preferably 250 nm or more, and more preferably 10 $\mu$m or less, still more preferably 1 $\mu$m or less, and particularly preferably 0.5 $\mu$m or less.

The size of the hollow portion refers to a length of a long axis of the hollow portion, and a diameter of the smallest circumscribed circle circumscribing the hollow portion observed by SEM or TEM is defined as the size of the hollow portion.

In the case where the hollow particles encapsulated in the light-scattering silica particles are too large, the number of light-scattering interfaces decrease and the scattering effect will be reduced. In the case where the hollow particles are too large, it is difficult to obtain spherical light-scattering silica particles, resulting in a poor sense of touch. The hollow particles preferably have a 50% particle diameter ($D_{50}$) of 200 nm to 50 $\mu$m. The 50% particle diameter ($D_{50}$) of the hollow particles is more preferably 230 nm or more, and still more preferably 250 nm or more. The 50% particle diameter ($D_{50}$) of the hollow particles is more preferably 10 $\mu$m or less, still more preferably 1 $\mu$m or less, and particularly preferably 0.5 $\mu$m or less.

The 50% particle diameter of the hollow particles is calculated using a laser diffraction particle size distribution analyzer (for example, MT3300EXII manufactured by MicrotracBEL Corp.).

In the case where the hollow portion-forming particles are template particles, the size of the template particle roughly matches the size of the hollow portion of the light-scattering silica particle, and therefore, the template particles preferably have a 50% particle diameter ($D_{50}$) in a range of 200 nm to 50 $\mu$m. The 50% particle diameter ($D_{50}$) of the template particles is more preferably 230 nm or more, still more preferably 250 nm or more, and more preferably 10 $\mu$m or less, still more preferably 1 $\mu$m or less, and particularly preferably 0.5 $\mu$m or less.

The 50% particle diameter of the template particles is calculated using a laser diffraction particle size distribution analyzer (for example, MT3300EXII manufactured by MicrotracBEL Corp.).

The hollow portion-forming particles may be spherical, or may have a shape other than the spherical shape, such as an ellipsoidal shape, or the hollow portion-forming particles may be partially united or aggregated.

The hollow portion-forming particles are preferably mixed in the silica precursor dispersion or silica precursor solution in a range of 0.1 mass% to 40 mass%. In the case where the amount of the hollow portion-forming particles used is too small, it may not be possible to encapsulate a plurality of hollow portions in the particles, and in the case where it is too large, the scattering of the light-scattering silica particles may become saturated, and further improvement of the light scattering effect may not be observed. By using the hollow portion-forming particles in a range of 40 mass% or less, the particles can be made light and the sense of touch can be improved, thereby improving the feeling during use.

The silica precursor dispersion or silica precursor solution may contain any other components. Examples of other components include sodium chloride, sodium sulfate, potassium chloride, and potassium sulfate, for the purpose of adjusting pore diameter distribution.

The organic phase used as the continuous phase preferably has low solubility in the silica precursor dispersion or silica precursor solution, and more preferably has no solubility in them.

Examples of an organic liquid constituting the organic phase include saturated linear hydrocarbons having 9 to 12 carbon atoms such as n-nonane, n-decane, n-undecane, and n-dodecane; saturated branched chain hydrocarbons having 9 to 12 carbon atoms such as isononane, isodecane, isoundecane, and isododecane; aliphatic hydrocarbons having 6 to 8 carbon atoms such as n-hexane, isohexane, n-heptane, isoheptane, n-octene, and isooctene; alicyclic hydrocarbons having 1 to 8 carbon atoms such as cyclopentane, cyclohexane, and cyclohexene; aromatic hydrocarbons such as benzene, toluene, xylene, ethylbenzene, propylbenzene, cumene, mesitylene, and tetralin; ethers such as propyl ether and isopropyl ether; esters such as ethyl acetate, n-propyl acetate, isopropyl acetate, n-putyl acetate, isobutyl acetate, n-amyl acetate, isoamyl acetate, butyl lactate, methyl propionate, ethyl propionate, butyl propionate, methyl butyrate, and ethyl butyrate and butyl butyrate; and fluorine-containing compounds such as 1,1,2,2-tetrafluoroethyl-2,2,2-trifluoroethyl ether.

These substances may be used alone or in combination of two or more kinds thereof. Among these, saturated linear hydrocarbons having 9 to 12 carbon atoms such as n-decane, n-nonane, n-dodecane, and n-undecane, and saturated branched hydrocarbons such as isononane, isodecane, isoundecane, and isododecane are preferred.

A flash point of the organic liquid constituting the organic phase is preferably 20°C to 80°C, and more preferably 30°C to 60°C. Non-flammable or flame-retardant organic liquids are preferred.

In the case where a saturated hydrocarbon with a flash point of less than 20°C is used as the organic liquid, since the flash point is low, measures must be taken for fire prevention and working environment. When the flash point of the organic liquid is 20°C or higher, it is possible to enhance a fire retardant property and improve the working environment. On the other hand, when the flash point is 80°C or lower, sufficient volatility can be obtained, and in the case where the light-scattering silica particles are recovered from the organic phase, it is possible to prevent the organic liquid from adhering to and mixing into the light-scattering silica particles.

A surfactant is added to the organic phase as an emulsifier. Examples of the surfactant include anionic surfactants, cationic surfactants, amphoteric surfactants, and nonionic surfactants.

Examples of the anionic surfactants include sodium fatty acids, alkyl sulfates, alkylbenzene sulfonates, and alkyl phosphates.

Examples of the cationic surfactants include alkyltrimethylammonium salts, dialkyldimethylammonium salts, and alkylbenzyldimethylammonium salts.

Examples of the amphoteric surfactants include alkyldimethylamine oxide, and alkylcarboxybetaine.

Examples of the nonionic surfactants include polyethylene glycol fatty acid ester, polypropylene glycol fatty acid ester, polyethylene glycol alkyl ether, sorbitan fatty acid ester, polyoxyethylene sorbitan fatty acid ester, polyoxyethylene alkylphenyl ether, and polyoxyethylene alkyl ether.

These surfactants may be used alone or in combination of two or more kinds thereof. Among these, sorbitan fatty acid esters such as sorbitan monooleate, sorbitan dioleate, and sorbitan trioleate are preferred.

An amount of the surfactant used can be appropriately selected depending on conditions such as the type of the surfactant, a hydrophile-lipophile balance (HLB), which is an index indicating a degree of hydrophilicity or hydrophobicity of the surfactant, and a target particle diameter of the silica particles. Specifically, the surfactant is preferably contained in the organic phase in an amount of 0.1 mass% to 5.0 mass%, more preferably 0.1 mass% to 3.0 mass%, and still more preferably 0.1 mass% to 1.0 mass%.

In the case where the surfactant content is 0.1 mass% or more in the organic phase, uniform emulsification can be achieved and the emulsion can be further stabilized. On the other hand, in the case where the surfactant content is 5.0 mass% or less, it is possible to prevent the surfactant from adhering to and mixing into the light-scattering silica particles that are the final product. In the case where a viscosity of the dispersed phase is low, a lower concentration of the surfactant is preferred, so as to achieve uniform emulsification.

The emulsion is obtained by adding the dispersed phase to the continuous phase and stirring the mixture. The dispersed phase is preferably added while stirring the continuous phase.

As a stirring method, a known method in the related art can be used, and an emulsification device such as a homogenizer, homomixer, colloid mill, ultrasonic emulsifier, and homodisperser can be used.

For example, when emulsifying using a homogenizer, stirring and emulsification may be performed under emulsification conditions under which a target droplet diameter can be achieved.

For example, when emulsifying using a homomixer, stirring and emulsification may be performed at a speed of 1,000 rpm to 24,000 rpm for 1 minute to 10 minutes.

A liquid temperature during stirring and emulsification is preferably 10°C to 40°C, more preferably 10°C to 35°C, and still more preferably 10°C to 30°C.

Next, the obtained emulsion is solidified and then silica is precipitated to obtain a silica slurry containing silica encapsulating the hollow portion-forming particles.

Examples of a method for solidifying include adding an acid under stirring. As a result, the pH of the silica precursor dispersion or the silica precursor solution decreases, and primary particles of silica are precipitated. Since the precipitated primary particles aggregate, secondary particles of silica encapsulating the hollow particles are generated. Conditions for adding the acid may be such that spherical particles are obtained at the end of gelling, and the pH of an aqueous phase after two-phase separation is around neutral, preferably pH 7 to 10.

The acid used may be either an inorganic acid or an organic acid, in which inorganic acids such as sulfuric acid, hydrochloric acid, and carbonic acid are more preferred, and carbon dioxide gas is the most preferred for ease of operation.

A reaction solution is separated into the aqueous phase in which silica encapsulating the hollow portion-forming particles is precipitated and an oil phase, and therefore, the oil phase is removed to obtain the silica slurry.

The oil phase can be removed by a known method in the related art, such as decantation, vacuum filtration, pressure filtration, membrane separation, and centrifugation.

Next, in the case where the hollow portion-forming particles are hollow particles, the obtained silica slurry is washed and dried. In the case where the hollow portion-forming particles are template particles, the template particles are removed, followed by cleaning and firing.

A case where the hollow portion-forming particles are hollow particles will be explained.

Examples of a cleaning liquid for cleaning the silica slurry include water and organic solvents such as ethanol, acetone, and diethyl ether.

A temperature of the cleaning liquid is preferably 10°C to 100°C, more preferably 20°C to 90°C, and still more preferably 30°C to 80°C.

The silica slurry is thoroughly cleaned, and then dried. The drying may be performed by a known method in the related art. Examples of the drying method include vacuum drying, natural drying, and hot air drying, and a spray dryer, flash dryer, tray dryer, vacuum dryer, kiln and the like can be used.

As for drying conditions, it is sufficient that the cleaning liquid can be removed and the product can be dried, and the drying conditions may be adjusted as appropriate depending on the drying method employed.

A case where the hollow portion-forming particles are template particles will be explained.

In the case where the template particles are used, in a step of producing light-scattering silica particles using hollow particles, after replacing the hollow particles with template particles to produce silica particles, the template particles are removed, followed by firing.

The template particles of the thermally decomposable type are heated to a temperature at which the template particles are thermally decomposed, and then a porous portion around the hollow portion is fired to make the pores nonporous. As a result, a periphery of the hollow portion becomes denser and a closed pore structure is obtained. As the firing method, known firing methods in the related art can be used, and an electric furnace, an oven, and the like can be used.

The temperature at which the template particles are thermally decomposed is not particularly limited as long as the template particles can be removed, and is preferably in a range of 300°C to 700°C. A thermal decomposition time is not particularly limited as long as the template particles can be removed, and can be freely set between 1 second and 24 hours, for example.

A firing temperature for crushing the pores around the hollow portion is preferably 800°C to 1300°C. By firing at a temperature of 800°C or higher, the silica around the hollow portion can be made dense, and by firing at a temperature of 1300°C or lower, sintering of particles during firing can be prevented. The firing temperature is more preferably 900°C or higher, still more preferably 1000°C or higher, and more preferably 1200°C or lower, and still more preferably 1100°C or lower. A firing time is not particularly limited as long as the silica around the hollow portion is densified and the hollow portion becomes a closed pore, and can be freely set between 1 second and 24 hours, taking into consideration the firing methods to be used and production efficiency.

Heating may be performed successively by heating for decomposing the template particles and heating for firing, and after removing the template particles by heating, refiring may be performed by reheating the silica particles once returned to room temperature.

Removal of acid-soluble template particles is performed by adding an acid to the silica slurry. Examples of acids that can be used include inorganic acids such as sulfuric acid, hydrochloric acid, and nitric acid, and organic acids such as acetic acid, perchloric acid, hydrobromic acid, trichloroacetic acid, dichloroacetic acid, methanesulfonic acid, and benzenesulfonic acid. Among them, it is preferable to use sulfuric acid from the viewpoint of safety and equipment protection.

The acid is preferably added until the pH of the silica slurry becomes 3 or less. As a result, the template particles are dissolved and removed from the silica, and voids derived from the template particles are formed inside the silica particles.

The pH is preferably 1.5 to 2.5, and more preferably 1.5 to 2.0.

After removing the template particles, the silica from which the template particles are removed is cleaned.

The cleaning liquid for cleaning the silica is the same as above, and examples thereof include water and organic solvents such as ethanol, acetone, and diethyl ether.

A temperature of the cleaning liquid is preferably 10°C to 100°C, more preferably 20°C to 90°C, and still more preferably 30°C to 80°C.

Then, the silica is thoroughly cleaned and then fired. By firing the silica, the periphery of the hollow portion becomes a dense layer and a closed pore structure is defined. As the firing method, known firing methods in the related art can be used, and an electric furnace, an oven, and the like can be used.

The firing temperature is the same as that when using thermally decomposable template particles, and is preferably 800°C to 1300°C. By firing at a temperature of 800°C or higher, the silica around the hollow portion can be made dense, and by firing at a temperature of 1300°C or lower, sintering of particles during firing can be prevented. The firing temperature is more preferably 900°C or higher, still more preferably 1000°C or higher, and more preferably 1200°C or lower, and still more preferably 1100°C or lower.

A firing time is not particularly limited as long as the silica around the hollow portion is densified and the hollow portion becomes a closed pore, and can be freely set between 1 second and 10 hours, taking into consideration the firing methods to be used and production efficiency.

In the present invention, the surfaces of the light-scattering silica particles may be modified. Surface modification may be performed during production of the light-scattering silica particles, specifically, after solidifying the emulsion to precipitate silica and before cleaning, or may be performed using the obtained light-scattering silica particles, and from the viewpoint of efficiency of modification, it is preferable to perform the modification in a state where no insoluble substances are attached to the particle surfaces.

For the surface modification of the light-scattering silica particles, for example, it is preferable to support one or two or more surface treatment agents selected from the group consisting of silicone, silylating agents, fatty acids having 12 or more carbon atoms and metal salts thereof, and higher alcohols having 14 or more carbon atoms on the particle surfaces. By supporting these surface treatment agents, hydrophobicity can be imparted to the light-scattering silica particles.

A method for surface modification using silicone, silylating agents, and fatty acids having 12 or more carbon atoms and metal salts thereof is not particularly limited, and any known method can be used. Examples thereof include a method of dry treating the surface treatment agent on the silica particles and a method of wet treating the surface treatment agent on the silica particles by immersing the silica particles in a solution in which a surface treatment agent is dissolved or dispersed in a solvent such as water or an organic compound.

Specifically, as a method for supporting silicone and a silylating agent on the light-scattering silica particles, for example, light-scattering silica particles coated with the surface treatment agent can be obtained by spraying the surface treatment agent diluted as necessary while stirring the silica particles, and then performing heating and mixing at 100°C to 400°C. Examples of other methods include a method in which steam generated by heating a surface treatment agent above a boiling point thereof is introduced into a fluidized bed or into silica particles under stirring.

As a method for supporting a fatty acid having 12 or more carbon atoms and a metal salt thereof on the light-scattering silica particles, for example, in a state where the light-scattering silica particles and the fatty acid are allowed to coexist in water or an organic solvent, a soluble metal salt such as aluminum chloride or zinc chloride is added to precipitate the metal salt of the fatty acid on the powder, to thereby obtain light-scattering silica particles coated with a surface treatment agent.

As a method for supporting a higher alcohol having 14 or more carbon atoms on the light-scattering silica particles, first, the light-scattering silica particles and the higher alcohol having 14 or more carbon atoms are mixed, and the higher alcohol having 14 or more carbon atoms is adhered to the surfaces of the particles.

Since the higher alcohol having 14 or more carbon atoms are solid or viscous liquid at room temperature, it is preferable to dissolve the higher alcohol having 14 or more carbon atoms or reduce the viscosity thereof by heating and then mix with the silica particles.

A reaction temperature and a reaction time in the mixing step can be appropriately set as long as the higher alcohol having 14 or more carbon atoms melts and is adhered to the silica particles.

After adhering the higher alcohol having 14 or more carbon atoms to the surfaces of the light-scattering silica particles, heat treatment is performed at a temperature of 160°C or higher to bond the higher alcohol having 14 or more carbon atoms to the surfaces of the silica particles.

A reaction temperature of the bonding step is more preferably 165°C or higher, and still more preferably 170°C or higher. Although an upper limit of the reaction temperature is not particularly limited, the reaction temperature is preferably 300°C or lower and more preferably 250°C or lower, from the viewpoint of preventing thermal decomposition of the higher alcohol having 14 or more carbon atoms as a raw material.

As a heating method, a known method in the related art may be used, and examples thereof include a method of heating with a heating device, such as the Henschel mixer, a double cone dryer, the Nauta mixer, or a vibration dryer. The heat treatment is preferably performed in an inert gas atmosphere or under reduced pressure.

The reaction time in the bonding step is preferably 2 hours to 8 hours. In the case where the reaction time is 2 hours or more, a condensation reaction between the light-scattering silica particles and the higher alcohol having 14 or more carbon atoms proceeds appropriately, and in the case where the reaction time is 8 hours or less, high productivity can be achieved.

The reaction time is more preferably 3 hours or more, and more preferably 87 hours or less.

By cooling after heating, the light-scattering silica particles supporting the higher alcohol having 14 or more carbon atoms can be obtained.

A mixing ratio of the light-scattering silica particles and the surface treatment agent is preferably such that the mixing is performed so that the supported amount of the surface treatment agent per unit specific area of the particles is 1 $\mu$mol/m$^2$ to 6 $\mu$mol/m$^2$. In the case where the supported amount of the surface treatment agent is 1 $\mu$mol/m$^2$ or more, light-scattering silica particles having sufficient hydrophobicity can be obtained. In the case where the amount of the surface treatment agent used is too large, a large amount of the surface treatment agent remains without adhering to the particles, and therefore, the amount is preferably 6 $\mu$mol/m$^2$ or less.

The surface treatment agent is more preferably mixed so that the supported amount thereof per unit specific area of the particles is 1.2 mol/m$^2$ or more, and still more preferably 1.5 $\mu$mol/m$^2$ or more, and is more preferably 5 $\mu$mol/m$^2$ or less, and still more preferably 4 $\mu$mol/m$^2$ or less.

The light-scattering silica particles of the present invention include a plurality of hollow portions each having a closed pore structure inside the spherical particle, and thus have a scattering property against light, and can exhibit light scattering properties for both visible light and ultraviolet rays when a water cake with a silica amount of 20 mg/cm$^2$ per measurement cross-sectional area is prepared.

In the case where hollow particles and solid particles are mixed separately in a liquid composition, a difference in filler concentration may occur in the composition, with the hollow particles floating and the solid particles settling. In order to obtain a light scattering effect, it is desirable to uniformly disperse the hollow particles in the composition, but the hollow particles may become localized due to aggregation and the like, and may not be able to fully exhibit the scattering properties thereof.

However, the light-scattering silica particles according to the present invention include the hollow portions each having a closed pore structure inside the spherical particle, and therefore can be uniformly dispersed in the composition.

The light-scattering silica particles according to the present invention can be used as fillers for cosmetics, white paints, UV-reflecting paints, light-diffusing films, films for membrane structures, films for agricultural greenhouses, thermosetting resins, ultraviolet curing resins, thermoplastic resins, and the like.

A cosmetic containing the light-scattering silica particles according to the present invention has a sunscreen effect, is free from the risk of component deterioration due to photocatalysts, and can be safely used on skin.

When the light-scattering silica particles according to the present invention are contained in an ultraviolet curing resin, since ultraviolet rays diffuse into an interior thereof, it is possible that the light-scattering silica particles according to the present invention are contained at a high concentration.

[Examples]

Hereinafter, the present invention will be described with reference to Examples, but the present invention is not limited thereto. In the following description, common components employ the same substance. Unless otherwise specified, "parts" and "%" represent "parts by mass" and "mass%" respectively.

Examples 2, 3, 7, 8, and 11 are working examples, and Examples 1, 4 to 6, 9, 10, 12, and 13 are comparative examples. Examples 8 to 13 are examples of application to cosmetic formulations.

(Example 1)

<Preparation of Emulsion>

To 1250 g of pure water, 7 g of EO-PO-EO block copolymer (Pluronic F68 manufactured by ADEKA CORPORATION) in which polyoxyethylene block (EO) and polyoxypropylene block (PO) were combined was added and stirred until dissolved. Crude emulsion was prepared by adding 42 g of n-dodecane to the resulting aqueous solution and stirring the resulting liquid using a homogenizer manufactured by IKA-Werke GmbH & Co. KG until the entire liquid becomes uniform.

Fine emulsion was produced by emulsifying this crude emulsion two times at the pressure of 400 bar using a high-pressure emulsifier (LAB2000 manufactured by SMT Co., Ltd.), and the fine emulsion was left standing at room temperature (25°C) for 15 hr.

<First Stage Shell Formation>

Into a glass reaction tank with a capacity of 2 L, 1300 g of the fine emulsion obtained above was added, and 41 g of a diluted aqueous sodium silicate solution ($SiO_2$ concentration 10.4%, $Na_2O$ concentration 3.6%) and 1 M sulfuric acid were added so that the pH became 2, and the resulting liquid was stirred well while being maintained at 40°C.

While the liquid was stirred well, 1 M sodium hydroxide aqueous solution was dropped slowly to this liquid so that the pH became 6, thereby obtaining oil core-silica shell particle dispersion. The oil core-silica shell particle dispersion thus obtained was held and matured.

<Second Stage Shell Formation>

The entire oil core-silica shell particle dispersion obtained by the first stage shell formation was heated to 70°C and 1 M NaOH was added slowly while the liquid was stirred so that the pH became 9.

Subsequently, 460 g of a diluted sodium silicate aqueous solution ($SiO_2$ concentration: 10.4%, $Na_2O$ concentration: 3.6%) and 0.25 M sulfuric acid were added gradually so that the pH became 9.

Thereafter, the resulting liquid was cooled to room temperature to obtain a hollow silica precursor dispersion.

<Filtration, Drying, Firing>

The entire hollow silica precursor dispersion was filtered by pressure filtration (pressure: 0.28 MPa) using 0.45 $\mu$m-hydrophilic PTFE membrane filter.

A cake obtained after the filtration was dried in a nitrogen atmosphere at 60°C for 1 hour and then at 400°C for 4 hours (temperature increase rate: 5°C/min) to remove organic components, thereby obtaining a hollow silica precursor.

Shell refiring was performed by firing the obtained precursor at 900°C for 4 hours (temperature increase rate: 5°C/min) to thereby obtain hollow silica particles (50% particle diameter: 1.6 $\mu$m, density measured using Ar: 0.98 g/cm$^3$, volume ratio of hollow portions: 55 volume%).

(Example 2)

A slurry, which is obtained by stirring and mixing 0.35 parts of the hollow silica particles (50% particle diameter: 1.6 $\mu$m) prepared in Example 1 with 100 parts of an aqueous solution that is obtained by adding 0.3 g/g-SiOz of sodium chloride into a solution obtained by diluting No. 3 sodium silicate (manufactured by AGC Si-Tech Co., Ltd., sodium silicate aqueous solution, $SiO_2/Na_2O$ (molar ratio) = 3) with distilled water so as to make the $SiO_2$ concentration 12%, was used as the dispersed phase. The amount of the hollow silica particles added to 100 parts of SiOz component in No. 3 sodium silicate was 3 parts.

A phase in which 0.7% of sorbitan monooleate (manufactured by Sanyo Chemical Industries, Ltd., IONET S80) as a surfactant was dissolved in n-decane (manufactured by Tosoh Corporation, HC-250, $C_{10}H_{22}$) in advance was used as the continuous phase.

While stirring the continuous phase, which is the surfactant-containing n-decane, with a homogenizer (manufactured by MICROTEC CO., LTD., Physcotron, generator shaft NS-30UG/20P), the dispersed phase, which is the sodium silicate slurry containing the hollow silica, was added and emulsified by stirring at 7,000 rpm for 5 minutes.

While stirring the obtained emulsion using a stirrer (manufactured by Tokyo Garasu Kikai Co., Ltd., Three-One Motor), carbon dioxide gas was blown into the emulsion at a rate of 2.0 L/min for 20 minutes to precipitate silica.

Thereafter, an aqueous phase containing silica gel obtained by removing an oil phase was cleaned by decantation, dried with a spray dryer (Mini Spray Dryer B290 manufactured by Nihon BUCHI K.K.), to thereby obtain the light-scattering silica particles (volume ratio (calculated value) of hollow portions: 0.7 volume%).

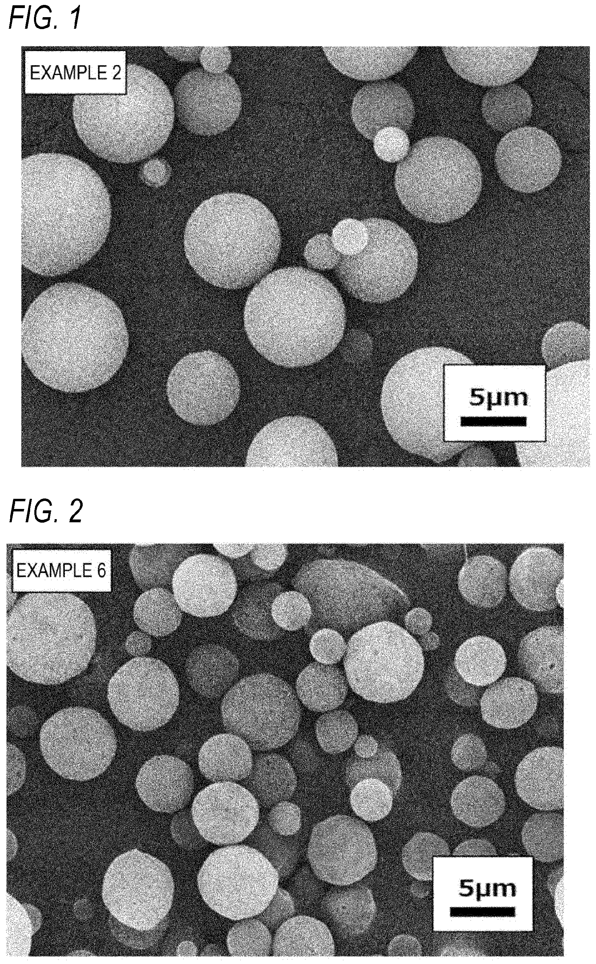

The obtained light-scattering silica particles were imaged using a scanning electron microscope (manufactured by JEOL Ltd., JCM-7000). The SEM image is shown in FIG. 1.

(Example 3)

Light-scattering silica particles (volume ratio (calculated value) of hollow portions: 1.3 volume%) were obtained in the same manner as in Example 2, except that 0.58 parts of the hollow silica particles were mixed in the preparation of the dispersed phase. Note that the amount of the hollow silica particles added to 100 parts of $SiO_2$ component in No. 3 sodium silicate was 5 parts.

(Example 4)

Commercially available porous spherical silica ("SUNSPHERE H-53" manufactured by AGC Si-Tech Co., Ltd.) was used.

(Example 5)

Commercially available porous spherical silica ("SUNSPHERE H-51" manufactured by AGC Si-Tech Co., Ltd.) was used.

(Example 6)

A slurry, which is obtained by stirring and mixing template particles made of magnesium hydroxide (manufactured by Konoshima Chemical Co.,Ltd., MAGSEEDS X-6F, 50% particle diameter: 0.8 $\mu$m) into a solution that is obtained by diluting No. 3 sodium silicate (manufactured by AGC Si-Tech Co., Ltd., sodium silicate aqueous solution, $SiO_2/Na_2O$ (molar ratio) = 3) with distilled water so as to make the $SiO_2$ concentration 20%, was used as the dispersed phase.

A phase in which 1.4% of sorbitan monooleate (manufactured by Sanyo Chemical Industries, Ltd., IONET S80) as a surfactant was dissolved in n-decane (manufactured by Tosoh Corporation, HC-250, $C_{10}H_{22}$) in advance, was used as the continuous phase.

While stirring the continuous phase, which is the surfactant-containing n-decane, with a homogenizer (manufactured by MICROTEC CO., LTD., Physcotron, generator shaft NS-30UG/20P), the dispersed phase, which is the sodium silicate slurry containing magnesium hydroxide, was added and emulsified by stirring at 12,000 rpm for 5 minutes.

While stirring the obtained emulsion using a stirrer (manufactured by Tokyo Garasu Kikai Co., Ltd., Three-One Motor), carbon dioxide gas was blown into the emulsion at a rate of 2.0 L/min for 20 minutes to precipitate silica.

Thereafter, while stirring the aqueous phase containing silica gel, which is obtained by removing the oil phase, at room temperature, sulfuric acid was added until the pH reached 2 to remove the template. Furthermore, the mixture was stirred for 30 minutes while maintaining the temperature at 80°C in a constant temperature aqueous phase and adjusting the pH to 2 with sulfuric acid to remove the template and residual solvent.

The slurry after removing the template and the solvent was cleaned by decantation and dried with a spray dryer (Mini Spray Dryer B290, manufactured by Nihon BUCHI K.K.) to thereby obtain silica particles.

The obtained silica particles were imaged using a scanning electron microscope (manufactured by JEOL Ltd., JCM-7000). The SEM image is shown in FIG. 2.

(Example 7)

Light-scattering silica particles whose surfaces were made hydrophobic were produced by the following production method.

Into a closed container, 10 g of the light-scattering silica particles produced in Example 3 and 4.1 g of behenyl alcohol ("Calcol 220-80" manufactured by Kao Corporation) were weighed out, and the closed container was placed in a hot water bath set at 95°C, followed by heating and mixing for 2 hours.

Next, 5 g of the heated mixture was weighed out and heated under reduced pressure at 180°C for 4 hours in a vacuum dryer (SVD-10P, SANSHO CO.,LTD.). Thereafter, the mixture was allowed to cool and collected to thereby obtain 4.7 g of hydrophobized light-scattering silica particles.

<Test Example 1>

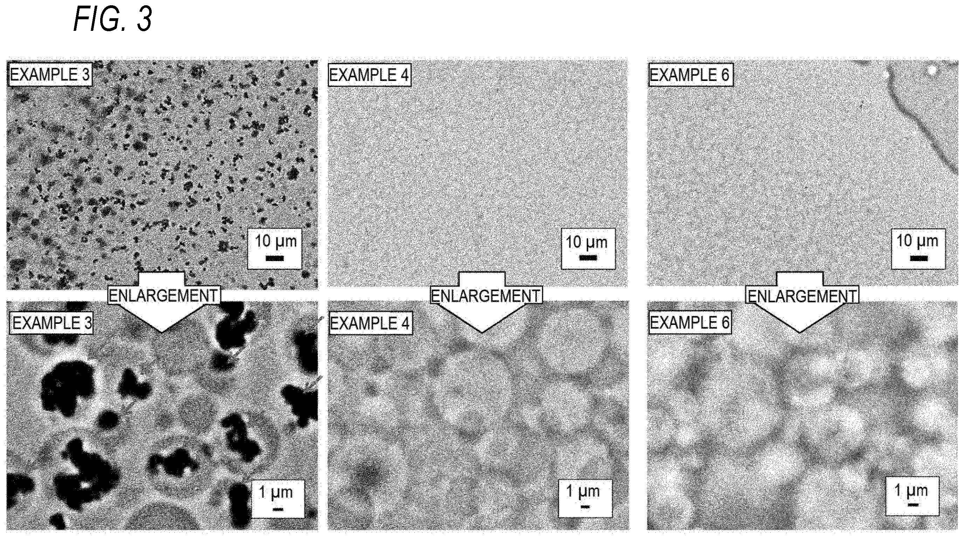

The following measurements were performed on the particles of Examples 1 to 6. Results are shown in Table 1 and FIGs. 3 to 5.

1. Reflectance

A reflectance of a water cake containing 20 mg/cm$^2$ of silica per measurement cross-sectional area was calculated as follows.

First, water was added to each silica powder in a unipack and mixed to produce a silica water cake with no fluidity. The amount of water added was 1.1 to 1.5 times the oil absorption value.

Next, the water cake was packed into a powder sample cell so that the amount of silica per measurement cross-sectional area was 20 mg/cm$^2$ to 30 mg/cm$^2$, and the reflectance (reflectance a) of the water cake at wavelengths of 310 nm and 600 nm was measured using an ultraviolet-visible light-near infrared spectrophotometer UV-3100PC (manufactured by Shimadzu Corporation). Thereafter, the reflectance (reflectance b) at wavelengths of 310 nm and 600 nm was measured without filling the cell with a sample. An interpolated value of the reflectance a and the reflectance b was calculated as a reflectance when the amount of silica per measurement cross-sectional area was 20 mg/cm$^2$.

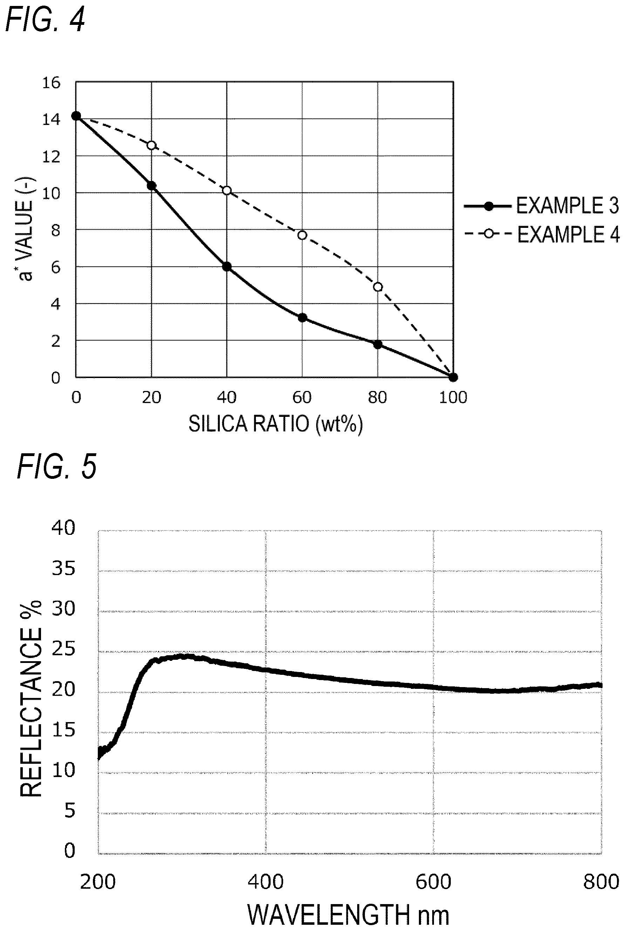

For the particles of Example 3, after preparing a 1% water slurry, the reflectance at wavelengths of 310 nm and 600 nm was measured using the ultraviolet-visible light-near infrared spectrophotometer UV-3100PC as described above. Results are shown in FIG. 5.

2. Presence or Absence of Hollow Portions of 200 nm to 50 $\mu$m inside Particles

The particle powder was immersed in boiled linseed oil and observed with an optical microscope (Morphologi 4, manufactured by Malvern Panalytical Ltd), and the case where scatterers with a size of 200 nm to 50 $\mu$m and a contrast different from surroundings were observed inside the particles was evaluated as "presence" of the hollow portions of 200 nm to 50 $\mu$m, and the case where the scatterers were not observed was evaluated as "absence" of the hollow portions of 200 nm to 50 $\mu$m.

Note that optical microscope images of the silica particles of Examples 3, 4, and 6 are shown in FIG. 3.

3. Volume Ratio of Hollow Portions of 200 nm to 50 $\mu$m inside Particles

The volume ratio (%) of the hollow portions inside the particles was determined as follows. The volume ratio of the hollow portions is expressed as a percentage obtained by dividing the total volume occupied by the hollow portions by a sum of a volume occupied by silica and a total volume occupied by the hollow portions.

The volume ratio was determined by calculation using a density of the silica particles measured using argon (Ar) gas and a true density (2.2 g/cm$^3$) of silica. The density measured using Ar gas was measured using Ar gas with a dry automatic density meter (AccuPyc II 1340, manufactured by Shimadzu Corporation).

Assuming that the density measured using Ar gas is $\rho$ (g/cm$^3$), the volume ratio of the hollow portions was determined using an equation 100 - 100 $\times$ $\rho$/2.2 (%).

4. Oil Absorption Value (cm$^3$/100 g)

The oil absorption value was measured in accordance with JIS K 5101 (2004).
Boiled linseed oil was added to the sample, which is the silica particles, while kneading until the entire sample became a lump, and a volume of the boiled linseed oil per 100 g of the sample was determined when the entire sample became a lump.

5. 50% particle diameter ($\mu$m)

The 50% volume equivalent particle diameter (50% particle diameter) was calculated using a laser diffraction particle size distribution analyzer (MT3300EXII manufactured by MicrotracBEL Corp.).

6. Specific Surface Area (m$^2$/g)

The specific surface area was determined by using an automatic specific surface area/pore distribution analyzer (TriStar II 3020 series manufactured by Shimadzu Corporation) and analyzing results measured by the nitrogen adsorption method at a relative pressure of 0 to 0.99 using the BET method.

7. Pore Volume (cm$^3$/g)

Same as the specific surface area, the pore volume was determined by using an automatic specific surface area/pore distribution analyzer (TriStar II 3020 series manufactured by Shimadzu Corporation) and analyzing results measured by the nitrogen adsorption method at a relative pressure of 0 to 0.99 using the BJH method.

8. Average Circularity (-)

The circularity was calculated by obtaining an area and a perimeter of a particle with an image obtained by a particle image analyzer (Morphologi 4 manufactured by Malvern Panalytical Ltd) by using image analysis software attached to the above analyzer and applying the results to the following equation. The average circularity of 30,000 silica particles was determined as the average circularity.

$$\text{Circularity} = \text{perimeter of circle with equal projected area/perimeter of particle}$$

Perimeter of circle with equal projected area: observing a particle from directly above, obtaining an area of a shadow of the particle reflected on a plane below, and calculating a circle having an area equal to this area, so as to obtain a length of an outline of the circle
Perimeter of particle: observing the particle from directly above to obtain a length of an outline of the shadow of the particle reflected on the plane below

9. Sense of Touch

An appropriate amount of the particles (half amount on a micro spatula) was applied to an inner side of an upper arm, and the sense of touch was checked with fingertips.
Those that did not feel squeak were rated "A (good)" and those that did feel squeak were rated "B (poor)".

10. Static Friction Coefficient, Dynamic Friction Coefficient (-)

The friction coefficients were obtained using a static and dynamic friction measuring machine "TL201Tt" (Manufactured by Trinity-Lab Inc.). A urethane artificial finger was used as a contactor, and a load was set to 30 gf, a scanning distance was set to 40 mm, and a scanning speed was set to 10 mm/sec, and artificial leather "sapurare" (manufactured by IDEATEX JAPAN Co., Ltd.) was used as a coating substrate, and the friction coefficients were measured with a coating amount of the silica particles in each Examples being 0.8 $\mu$L/cm$^2$ in terms of the bulk volume per unit area. Among the obtained friction coefficients, an average value in a range from 1,000 msec to 4,000 msec was employed as the dynamic friction coefficient.
The static friction coefficient was obtained from the maximum value in a range from 0 msec to 1,000 msec.

11. a' value (-)

The a' values were measured for the particles of Example 3 and Example 4.

Iron (III) oxide powder (manufactured by KANTO CHEMICAL Co., INC., Guaranteed reagent, reddish brown powder, a' value: 14.9) was added to each particle powder at a ratio of 20, 40, 60 or 80% and mixed well to obtain a mixed powder. The mixed powder was placed in a round cell for measurement (diameter 35 mm $\times$ height 15 mm) to half or more of the volume, and measured using a spectrocolorimeter (manufactured by NIPPON DENSHOKU INDUSTRIES CO., LTD., SE7700).

Note that the a' value indicates strength of a color, and the higher the value is on the + side, the stronger the redness, and the higher the value is on the - side, the stronger the greenness. In the case where iron (III) oxide powder was mixed at a ratio of 40%, when the a' value is 6.0 or less, it can be determined that the color (redness) of iron (III) oxide is reduced.

Results are shown in FIG. 4.

[Table 1]

|  | Amount (parts) of hollow particles added | Reflectance (%R) on water cake when silica amount was 20 mg/cm$^2$ | | | Presence or absence of hollow portions of 200 nm to 50 $\mu$m | Specific surface area (m$^2$/g) | Pore volume (cm$^3$/g) |
|---|---|---|---|---|---|---|---|
|  |  | 310 nm (A) | 600 nm (B) | (A) - (B) |  |  |  |
| Example 1 | 100 | 89.7 | 91.0 | -1.3 | Presence | 39 | 0.09 |
| Example 2 | 3 | 47.6 | 38.2 | 9.4 | Presence | 606 | 1.23 |
| Example 3 | 5 | 52.6 | 43.8 | 8.8 | Presence | 651 | 1.33 |
| Example 4 | 0 | 27.6 | 26.0 | 1.6 | Absence | 701 | 1.94 |
| Example 5 | 0 | 22.7 | 22.2 | 0.5 | Absence | 712 | 0.76 |
| Example 6 | 0 | 21.8 | 20.8 | 1.0 | Presence | 791 | 1.40 |

[Table 1] (continued)

|  | Oil absorption value (cm$^3$/100 g) | 50% particle diameter ($\mu$m) | Shape | Average circularity | Sense of touch | Static friction coefficient | Dynamic friction coefficient |
|---|---|---|---|---|---|---|---|
| Example 1 | 403 | 1.6 (primary particle: 0.3 $\mu$m) | Aggregates of spherical particles | 0.70 | B | 0.73 | 0.45 |
| Example 2 | 336 | 6.1 | Spherical | 0.99 | A | 0.39 | 0.24 |
| Example 3 | 312 | 6.7 | Spherical | 0.99 | A | 0.40 | 0.26 |
| Example 4 | 348 | 7.4 | Spherical | 0.99 | A | 0.37 | 0.14 |
| Example 5 | 140 | 5.1 | Spherical | 0.99 | A | 0.42 | 0.18 |

(continued)

|  | Oil absorption value (cm$^3$/ 100 g) | 50% particle diameter (μm) | Shape | Average circularity | Sense of touch | Static friction coefficient | Dynamic friction coefficient |
|---|---|---|---|---|---|---|---|
| Example 6 | 290 | 7.0 | Spherical | 0.99 | A | - | - |

According to the results shown in Table 1 and FIGs. 1 to 3, it was found that the light-scattering silica particles of Examples 2 and 3 include hollow portions each having a closed pore structure inside, and have a light scattering property for both visible light and ultraviolet rays when measured with a water cake.

The light-scattering silica particles of Example 3 exhibited a reflectance A of 52.6% immediately after the water cake was prepared (see Table 1), and 51.0% even after being kept for one week in a state where water did not volatilize. The light-scattering silica particles of Example 3 exhibited a reflectance B of 43.8% immediately after the water cake was prepared (see Table 1), and 42.1% even after being kept for one week. When the light scattering property of the light-scattering silica particles of Example 3 was measured in a 1% water slurry using the above method, the reflectance A thereof was 24.4% and the reflectance B thereof was 20.6% (see FIG. 5). Even after being kept for one week in the 1% water slurry state, the reflectance A was approximately 23%, and the reflectance B was approximately 20%.

Accordingly, it can be said that even in a dry state, the light-scattering silica particles have the light scattering property for both visible light and ultraviolet rays, and as shown in FIG. 4, it was found that dry powder also has the light scattering property.

There was a clear difference in sense of touch between the hollow silica particles (Example 1) and other particles (Examples 2 to 6), and there were also differences in the dynamic friction coefficient and the static friction coefficient. It was found that the light-scattering silica particles of Examples 2 and 3 had a smooth sense of touch and were also excellent in feeling during use.

<Test Example 2>

The following measurements were performed on the particles of Examples 3 and 7. Results are shown in Table 2.

12. Water Repellency (-)

To a beaker (10 mL capacity) weighed with 8 g of water were gently added 0.05 g of weighed particles. Then, the particles were sufficiently spread on the surface of the water by tapping 20 times, and left still at room temperature in that state.

The solution was visually observed every day to evaluate states of suspended and settled particles in the solution. The case where the particles were suspended or settled one day after standing was evaluated as "C", and the case where the particles were suspended or settled 2 to 6 days after standing was evaluated as "B", and the case where the particles were not suspended or settled even after 7 days was evaluated as "A". It is determined that "B" has better water repellency than "C", and "A" has better water repellency than "B".

13. Stability in Oil (-)

Into to the same container (13.5 mL transparent bottle with a lid), 4 mL of water and 4 mL of liquid paraffin (product code: 122-04775, FUJIFILM Wako Pure Chemical Corporation) were weighed out, and 0.05 g of the particles was added to the two-layer solution, followed by shaking 20 times. Then, the resulting mixture was left to stand at room temperature.

The mixture was visually observed every day and presence or absence of settled particles in the aqueous phase was evaluated. The case where the particles were settled in the aqueous phase one day after standing was evaluated as "C", and the case where the particles were settled in the aqueous phase 2 to 6 days after standing was evaluated as "B", and the case where the particles were not settled in the aqueous phase even after 7 days was evaluated as "A". It is determined that "B" has better stability in oil than "C", and "A" has better stability in oil than "B".

[Table 2]

|  | Water repellency (-) | Stability in oil (-) |
|---|---|---|
| Example 3 | C | C |

(continued)

| | Water repellency (-) | Stability in oil (-) |
|---|---|---|
| Example 7 | A | A |

According to the results in Table 2, it was found that the hydrophobized light-scattering silica particles (Example 7) exhibited sufficient hydrophobicity and were excellent in water repellency and stability in oil.

(Examples 8 to 10)

Powdered face powders were prepared according to the compositions shown in Table 3 below.

(Production Method)

A powdered face powder was obtained by mixing each component three times for 30 seconds until uniform with a Henschel mixer (manufactured by HANIL ELECTRIC CO., LTD, Model LM-110T).

[Table 3]

| (Blending amount: mass%) | | | |
|---|---|---|---|
| Components | Example 8 | Example 9 | Example 10 |
| Hydrogen dimethicone treated talc (*1) | 89.59 | 89.59 | 94.59 |
| Zinc laurate (*2) | 5.00 | 5.00 | 5.00 |
| Silica particles of Example 3 | 5.00 | - | - |
| Silica particles of Example 4 | - | 5.00 | - |
| Yellow iron oxide (*3) | 0.30 | 0.30 | 0.30 |
| Red iron oxide (*4) | 0.10 | 0.10 | 0.10 |
| Black iron oxide (*5) | 0.01 | 0.01 | 0.01 |
| Total | 100 | 100 | 100 |

Details of each component in Table 3 are as follows.

(*1) Hydrogen dimethicone treated talc: Talc DN-SH (Dainihonkasei Co., Ltd.)
(*2) Zinc laurate: Powder base L (NOF Corporation)
(*3) Yellow iron oxide: SunPURO Yellow Iron Oxide C339001 (Sun Chemical Corp.)
(*4) Red iron oxide: SunPURO Red Iron Oxide C338021 (Sun Chemical Corp.)
(*5) Black iron oxide: SunPURO Black Iron Oxide C337001 (Sun Chemical Corp.)

(Examples 11 to 13)

Water-in-oil BB creams were prepared according to the compositions shown in Table 4 below. BB cream is an abbreviation for Blemish Balm or Beauty Balm, and is an all-in-one cosmetic product that serves as a serum, moisturizing cream, base makeup, foundation, and sunscreen.

(Production Method)

1) Components of Group A were mixed at 700 rpm for 3 minutes so as to be uniform using a disperser mixer (manufactured by PRIMIX Corporation, LABOLUTION, Model: Homo Disperser 2.5) to obtain a Group A mixture.
2) Components of Group B were mixed using a disperser mixer at 700 rpm for 3 minutes so as to be uniform to obtain a Group B mixture, followed by mixing with the Group A mixture to obtain a first composition.
3) Components of Group C were mixed three times for 30 seconds so as to be uniform using a Henschel mixer to obtain a Group C mixture, followed by mixing with the first composition to obtain a second composition.

4) Components of Group D were mixed so as to be uniform using a disperser mixer at 700 rpm for 3 minutes to obtain a Group D mixture.

5) While stirring the second composition at 5,000 rpm using a disperser mixer, the Group D mixture was added, followed by emulsification for 3 minutes to thereby obtain a water-in-oil BB cream.

[Table 4]

| | Components | Example 11 | Example 12 | Example 13 |
|---|---|---|---|---|
| | (Blending amount: mass%) | | | |
| A | Cyclopentasiloxane (*6) | 6.00 | 6.00 | 6.00 |
| | Isotridecyl isononanoate (*7) | 5.00 | 5.00 | 5.00 |
| | Octyl methoxycinnamate (*8) | 5.00 | 5.00 | 5.00 |
| | Hydrogenated polyisobutene (*9) | 4.00 | 4.00 | 4.00 |
| | (PEG-12 dimethicone/PPG-20) crosspolymer, caprylyl methicone (*10) | 3.00 | 3.00 | 3.00 |
| | Lauryl PEG-10 tris(trimethylsiloxy)silylethyl dimethicone (*11) | 2.00 | 2.00 | 2.00 |
| | Sorbitan sesquiisostearate (*12) | 1.00 | 1.00 | 1.00 |
| B | Cyclopentasiloxane (*6) | 7.50 | 7.50 | 7.50 |
| | Polyglyceryl-2 isostearate (*13) | 1.00 | 1.00 | 1.00 |
| | Quaternium-18 bentonite (*14) | 1.50 | 1.50 | 1.50 |
| C | Hydrogen dimethicone treated talc (*15) | 1.75 | 1.75 | 1.75 |
| | Titanium oxide, Al hydroxide, stearic acid (*16) | 5.00 | 5.00 | 5.00 |
| | Hydrogen dimethicone treated titanium oxide (*17) | 5.00 | 5.00 | 5.00 |
| | Yellow iron oxide (*18) | 1.00 | 1.00 | 1.00 |
| | Red iron oxide (*19) | 0.15 | 0.15 | 0.15 |
| | Black iron oxide (*20) | 0.10 | 0.10 | 0.10 |
| | Silica particles of Example 3 | 2.00 | - | - |
| | Silica particles of Example 4 | - | 2.00 | - |
| D | Water | 44.00 | 44.00 | 46.00 |
| | Butylene glycol (*21) | 3.00 | 3.00 | 3.00 |
| | PEG/PPG/Polybutylene glycol-8/5/3 glycerin (*22) | 1.00 | 1.00 | 1.00 |
| | Na chloride (*23) | 0.50 | 0.50 | 0.50 |
| | Phenoxyethanol (*24) | 0.50 | 0.50 | 0.50 |
| | Total | 100 | 100 | 100 |

Details of each component in Table 4 are as follows.

(*6) Cyclopentasiloxane: KF-995 (Shin-Etsu Chemical Co., Ltd.)
(*7) Isotridecyl isononanoate: Saracos 913 (The Nisshin OilliO Group, Ltd.)
(*8) Octyl methoxycinnamate: Nomcoat TAB (The Nisshin OilliO Group, Ltd.)
(*9) Hydrogenated polyisobutene: PARLEAM 4 (NOF Corporation)
(*10) (PEG-12 dimethicone/PPG-20) crosspolymer, caprylyl methicone: DOWSIL DC EL-7040 Hydro Elastomer Blend (Dow Chemical Japan Limited)
(*11) Lauryl PEG-10 tris(trimethylsiloxy)silylethyl dimethicone: DOWSIL ES-5300 Formulation Aid (Dow Chemical Japan Limited)
(*12) Sorbitan sesquiisostearate: Cosmol 182V (The Nisshin OilliO Group, Ltd.)

(*13) Polyglyceryl-2 isostearate: Cosmol 41V (The Nisshin OilliO Group, Ltd.)

(*14) Quaternium-18 bentonite: S-Ben W (HOJUN Co., Ltd.)

(*15) Hydrogen dimethicone treated talc: Talc DN-SH (Dainihonkasei Co., Ltd.)

(*16) Titanium oxide, Al hydroxide, stearic acid: ST-705SA (Titan Kogyo, Ltd.)

(*17) Hydrogen dimethicone treated titanium oxide: Titanium DN-SH (2) (Dainihonkasei Co., Ltd.)

(*18) Yellow iron oxide: SunPURO Yellow Iron Oxide C339001 (Sun Chemical Corp.)

(*19) Red iron oxide: SunPURO Red Iron Oxide C338021 (Sun Chemical Corp.)

(*20) Black iron oxide: SunPURO Black Iron Oxide C337001 (Sun Chemical Corp.)

(*21) Butylene glycol: 1,3-butylene glycol (Daicel Corporation)

(*22) PEG/PPG/Polybutylene glycol-8/5/3 glycerin: WILBRIDE S-753 (NOF Corporation)

(*23) Chloride Na: Sodium chloride (KANTO CHEMICAL CO.,INC.)

(*24) Phenoxyethanol: Phenoxyethanol S (Yokkaichi Chemical Company Limited)

The above PEG is polyethylene glycol and PPG is polypropylene glycol.

<Test Example 3>

A color difference ΔE was measured for the powdered face powders of Examples 8 to 10, and concealing properties for freckles of the water-in-oil BB creams of Examples 11 to 13 were evaluated. Results are shown in Table 5.

14. Color Difference ΔE (-)

To 0.5 g of powdered face powder, 200 μL of simulated sebum was added and mixed. Here, the simulated sebum is a mixture of tri(caprylic/glyceryl caprate)/octyldodecyl myristate/oleic acid/squalane = 33.3%/33.3%/20.0%/13.4%.

Tri(caprylic/glyceryl caprate): O. D. O (The Nisshin OilliO Group, Ltd.)
Octyldodecyl myristate: EXCEPARL OD-M (Kao Corporation)
Oleic acid: Lunac O-V (Kao Corporation)
Squalane: Phytosqualane (SOPHIM)

The color change of the obtained powder was measured using a colorimeter (ZE 6000, NIPPON DENSHOKU INDUSTRIES CO., LTD.) and evaluated based on a color difference ΔE from a face powder to which no simulated sebum was added.

It is determined that the smaller ΔE is, the more reduced a color change is when the product comes into contact with sebum on actual skin.

15. Concealing Property for Freckles (-)

On a freckled plate (FR-40, manufactured by Beaulax Co., Ltd.), the BB cream was applied in an amount of 2.5 mg/cm$^2$ and dried thoroughly. Then, the concealing property for the freckles was checked, and a case where freckles were difficult to see was evaluated as "A", and a case where freckles were faded was evaluated as "B", and a case where freckles were clearly visible was evaluated as "C".

It is determined that "B" is better than "C" and "A" is better than "B" in that appearance defects such as freckles on actual skin can be hidden.

[Table 5]

|  | Form | Silica particles | Color difference ΔE (-) | Concealing property for freckles (-) |
|---|---|---|---|---|
| Example 8 | Powdered face powder | Silica particles of Example 3 | 18 |  |
| Example 9 |  | Silica particles of Example 4 | 19 |  |
| Example 10 |  | None | 33 |  |

(continued)

|  | Form | Silica particles | Color difference ΔE (-) | Concealing property for freckles (-) |
|---|---|---|---|---|
| Example 11 | Water-in-oil BB cream | Silica particles of Example 3 | | A |
| Example 12 | | Silica particles of Example 4 | | B |
| Example 13 | | None | | C |

According to results in Table 5, it is determined that the powdered face powder (Example 8) containing the light-scattering silica particles of Example 3 has a low ΔE and can reduce the color change when it comes into contact with sebum on actual skin. It was also found that the BB cream (Example 11) containing the light-scattering silica particles of Example 3 has a concealing property for freckles evaluated as A, and is expected to be able to conceal appearance defects such as freckles on actual skin.

Although the present invention has been described in detail with reference to specific embodiments, it is apparent to those skilled in the art that various changes and modifications can be made without departing from the spirit and scope of the present invention. The present application is based on a Japanese Patent Application (No. 2021-113071) filed on July 7, 2021, the contents of which are incorporated herein by reference.

**Claims**

1. Light-scattering silica particles having a light scattering property, the light-scattering silica particles comprising:

   a plurality of hollow portions each having a closed pore structure inside the particle, wherein
   the light-scattering silica particles have a volume-based cumulative 50% particle diameter of 1 $\mu$m to 500 $\mu$m and an average circularity of 0.80 or more, and
   in use of the light-scattering silica particles, a reflectance A of a water cake containing 20 mg/cm$^2$ of silica per measurement cross-sectional area at an ultraviolet wavelength of 310 nm is 30% or more.

2. The light-scattering silica particles according to claim 1, wherein
   the light-scattering silica particles have an oil absorption value of 100 cm$^3$/100 g or more.

3. The light-scattering silica particles according to claim 1 or 2, wherein
   the hollow portions are defined by hollow particles.

4. The light-scattering silica particles according to any one of claims 1 to 3, wherein
   each of the hollow portions have a size of 200 nm to 50 $\mu$m.

5. The light-scattering silica particles according to any one of claims 1 to 4, wherein
   in use of the light-scattering silica particles, a reflectance B of a water cake containing 20 mg/cm$^2$ of silica per measurement cross-sectional area at a visible light wavelength of 600 nm is 30% or more.

6. The light-scattering silica particles according to claim 5, wherein
   a difference (A - B) obtained by subtracting the reflectance B from the reflectance A is 3% or more.

7. The light-scattering silica particles according to any one of claims 1 to 6, wherein
   one or two or more kinds selected from the group consisting of silicone, a silylating agent, a fatty acid having 12 or more carbon atoms and metal salts thereof, and a higher alcohol having 14 or more carbon atoms is supported on surfaces of the light-scattering silica particles.

8. A method for producing light-scattering silica particles having a light scattering property, the method comprising:

   dispersing hollow portion-forming particles in a silica precursor dispersion or a silica precursor solution;

performing an emulsification treatment to obtain droplets; and
solidifying the droplets.

9. The method for producing light-scattering silica particles according to claim 8, wherein
the hollow portion-forming particles are dispersed in the silica precursor dispersion or the silica precursor solution
in a range of 0.1 mass% to 40 mass%.

10. The method for producing light-scattering silica particles according to claim 8 or 9, wherein
the hollow portion-forming particles are hollow particles.

11. The method for producing light-scattering silica particles according to claim 10, wherein
the hollow particles are at least one of hollow silica particles and glass balloons.

12. The method for producing light-scattering silica particles according to claim 10 or 11, wherein
a hollow portion inside each of the hollow particles has a size of 200 nm to 50 $\mu$m.

13. The method for producing light-scattering silica particles according to claim 8 or 9, wherein
the hollow portion-forming particles are template particles, and after the droplets are solidified, the template particles
are removed, followed by firing at 800°C to 1300°C.

14. The method for producing light-scattering silica particles according to claim 13, wherein
the template particles have a size of 200 nm to 50 $\mu$m.

15. The method for producing light-scattering silica particles according to any one of claims 8 to 14, wherein
one or two or more kinds selected from the group consisting of silicone, a silylating agent, a fatty acid having 12 or
more carbon atoms and metal salts thereof, and a higher alcohol having 14 or more carbon atoms is attached to
surfaces of particles obtained by solidifying the droplets.

16. A cosmetic containing the light-scattering silica particles according to any one of claims 1 to 7.

*FIG. 1*

*FIG. 2*

FIG. 3

EP 4 368 573 A1

*FIG. 4*

*FIG. 5*

## INTERNATIONAL SEARCH REPORT

| International application No. |
|---|
| **PCT/JP2022/026087** |

### A. CLASSIFICATION OF SUBJECT MATTER

*C01B 33/193*(2006.01)i; *A61Q 17/04*(2006.01)i; *A61K 8/25*(2006.01)i
FI: C01B33/193; A61K8/25; A61Q17/04

According to International Patent Classification (IPC) or to both national classification and IPC

### B. FIELDS SEARCHED

Minimum documentation searched (classification system followed by classification symbols)

C01B33/193; A61Q17/04; A61K8/25

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2019/189020 A1 (AGC INC) 03 October 2019 (2019-10-03) claims 1, 4, 10, 13, paragraphs [0020], [0024] ,[0038], [0043]-[0044], [0052] | 1-2, 4-6, 8-9, 13-14, 16 |
| Y | | 1-7, 10-12, 15-16 |
| X | JP 2014-009146 A (SAKAI CHEM IND CO LTD) 20 January 2014 (2014-01-20) claim 1, paragraphs [0032], [0049], [0054], table 1, examples 3-4 | 1-2, 4-7, 16 |
| Y | | 1-7, 15-16 |
| X | JP 2021-075438 A (NIPPON STEEL CHEMICAL & MAT CO LTD) 20 May 2021 (2021-05-20) claims 1, 5-6, 12, paragraph [0029] | 1-2, 4-6 |
| Y | | 7 |
| X | WO 2017/183657 A1 (KAO CORPORATION) 26 October 2017 (2017-10-26) claims 9-18, paragraph [0045] | 1-2, 5-6, 16 |
| Y | | 1-7, 16 |

☑ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **09 August 2022** | **30 August 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
| --- |
| **PCT/JP2022/026087** |

**C.    DOCUMENTS CONSIDERED TO BE RELEVANT**

| Category* | Citation of document, with indication, where appropriate, of the relevant passages | Relevant to claim No. |
| --- | --- | --- |
| X | JP 2013-136469 A (TAIHEIYO MATERIALS CORP) 11 July 2013 (2013-07-11) claims 1, 4-5, table 3 | 1-6, 16 |
| Y | | 7 |
| Y | WO 2016/152872 A1 (ASAHI GLASS CO LTD) 29 September 2016 (2016-09-29) paragraphs [0056], [0063] | 7, 15 |
| Y | JP 2009-249249 A (KAO CORPORATION) 29 October 2009 (2009-10-29) claims 1, 3, paragraphs [0011], [0013] | 10-12 |
| A | WO 2012/147812 A1 (TOKUYAMA CORPORATION) 01 November 2012 (2012-11-01) entire text | 1-16 |

Form PCT/ISA/210 (second sheet) (January 2015)

INTERNATIONAL SEARCH REPORT
Information on patent family members

International application No.

**PCT/JP2022/026087**

| Patent document cited in search report | | | Publication date (day/month/year) | Patent family member(s) | Publication date (day/month/year) |
|---|---|---|---|---|---|
| WO | 2019/189020 | A1 | 03 October 2019 | JP 2021-100892 A claims 1, 11, 14, 17, paragraphs [0024], [0028], [0039], [0044]-[0045], [0053] | |
| JP | 2014-009146 | A | 20 January 2014 | (Family: none) | |
| JP | 2021-075438 | A | 20 May 2021 | (Family: none) | |
| WO | 2017/183657 | A1 | 26 October 2017 | CN 109071239 A claims 9-18, paragraph [0076] KR 10-2018-0134848 A JP 2017-193462 A | |
| JP | 2013-136469 | A | 11 July 2013 | (Family: none) | |
| WO | 2016/152872 | A1 | 29 September 2016 | JP 2018-83759 A paragraph [0056] | |
| JP | 2009-249249 | A | 29 October 2009 | (Family: none) | |
| WO | 2012/147812 | A1 | 01 November 2012 | US 2014/0057111 A1 entire text EP 2703348 A1 CN 103476707 A KR 10-2014-0005177 A | |

Form PCT/ISA/210 (patent family annex) (January 2015)

**EP 4 368 573 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H11322324 A

- JP 2021113071 A